Europäisches Patentamt

European Patent Office

Office européen des brevets

(11) Numéro de publication: **0 165 120**

**A1**

(12) # DEMANDE DE BREVET EUROPEEN

(21) Numéro de dépôt: **85400906.5**

(22) Date de dépôt: **09.05.85**

(51) Int. Cl.⁴: **C 12 N 5/00**
C 12 N 7/00, G 01 N 33/569
G 01 N 33/531

(30) Priorité: **09.05.84 FR 8407151**

(43) Date de publication de la demande:
**18.12.85 Bulletin 85/51**

(84) Etats contractants désignés:
**AT BE CH DE GB IT LI LU NL SE**

(71) Demandeur: **INSTITUT PASTEUR**
**25/28, rue du Docteur Roux**
**F-75015 Paris(FR)**

(71) Demandeur: **CENTRE NATIONAL DE LA RECHERCHE SCIENTIFIQUE (C.N.R.S.)**
**15, Quai Anatole France**
**F-75007 Paris(FR)**

(72) Inventeur: **Montagnier, Luc**
**21, rue de Malabry**
**F-92350 Le Plessis Robinson(FR)**

(72) Inventeur: **Chamaret, Solange**
**324, rue Lecourbe**
**F-75015 Paris(FR)**

(72) Inventeur: **Gruest, Jacqueline**
**6, rue du Gué**
**F-94240 L'Hay Les Roses(FR)**

(74) Mandataire: **Gutmann, Ernest et al,**
**S.C. Ernest Gutmann - Yves Plasseraud 67, boulevard Haussmann**
**F-75008 Paris(FR)**

(54) **Rétrovirus associé aux lymphadénopathies et adapté à des lignées continues de cellules lymphoblastoides B, capables de produire en continu ledit rétrovirus et procédé pour leur obtention.**

(57) L'invention concerne des lignées continues et permanentes de cellules caractérisées par leur capacité à produire un continu un rétrovirus ayant les caractéristiques antigéniques essentielles du virus considéré comme responsable du syndrome d'immunodéfricience acquise (SIDA). Ces lignées sont costituées par des cellules lymphoblastoides B infectables notamment par le rétrovirus LAV, préalablement adapté à des lymphocytes B, en présence de lymphocytes T.

L'invention concerne également les rétrovirus LAV adaptés aux susdites lignées continues et permanentes. Leurs extraits sont mis en oeuvre dans des tests de diagnostic *in vitro* du SIDA.

EP 0 165 120 A1

RETROVIRUS ASSOCIE AUX LYMPHADENOPATHIES ET ADAPTE A DES
LIGNEES CONTINUES DE CELLULES LYMPHOBLASTOIDES B, CAPABLES
DE PRODUIRE EN CONTINU LEDIT RETROVIRUS ET PROCEDE POUR
LEUR OBTENTION

L'invention concerne un rétrovirus associé aux
lymphadénopathies, ci-après dénommé rétrovirus LAV (abréviation de l'expression anglaise "Lymphadenopathy Associated Virus") ce rétrovirus étant adapté à des lymphocytes B et capable d'être produit en continu par des
lignées continues de cellules lymphablastoïdes B poussant
en suspension. Elle est plus particulièrement relative à
des lignées continues de cellules lymphablastoïdes B
capables de répliquer un rétrovirus ayant les caractéristiques antigéniques du virus LAV et à un procédé de production de telles lignées cellulaires.

L'invention concerne naturellement encore les
extraits susceptibles d'être obtenus à partir de tels
virus, plus particulièrement ceux qui sont susceptibles
d'être reconnus par les sérums de patients affectés par
le syndrome d'immuno-déficience acquise (SIDA) ou par des
syndromes mettant en jeu des lymphadénopathies, ou d'autres signes précurseurs du SIDA, ci-après désignés par
l'abréviation SLA. De ce fait elle concerne toutes les
applications possibles de ce virus et de ces extraits,
notamment celles destinées à la détection de la présence
de ce virus dans des échantillons biologiques, et plus
particulièrement au diagnostic in vitro des SLA et du
SIDA.

Le SIDA est une affection qui s'est récemment développée dans plusieurs pays. Il a été constaté que le
SIDA était souvent accompagné ou précédé de syndromes
lymphadénopathiques reconnaissables, à l'occasion de
l'étude histologique des nodules lymphatiques à des patients
qui en sont affectés. Ces lymphadénopathies précèdent ou
peut-être constituent une forme moins grave de la maladie, qui, dans son stade final, se manifeste par un effondrement des défenses immunitaires du sujet accompagné par l'apparition d'un sarcome de Kaposi ou des infections opportunistes.

L'isolement d'un rétrovirus considéré comme l'agent étiologique majeur du SIDA et des SLA a été décrit par S. BARRE-SINOUSSI et Coll. dans un article intitulé "Isolation of a T-lymphotropic retrovirus from a patient at risk for acquired immune deficiency syndrom (AIDS)", Science 220, 868-70 (1983). Ce rétrovirus a été isolé à partir d'une culture de lymphocytes T obtenue à partir d'un nodule lymphatique d'un homosexuel présentant un SLA. Des isolats de virus ont, depuis, été isolés à partir de nodules lymphatiques obtenus à partir d'autres personnes souffrant du SIDA, parmi lesquelles un hémophile B.

On rappellera ci-après les caractéristiques principales de ce rétrovirus :

- les cibles préférées de ce rétrovirus sont constituées par des lymphocytes T, plus particulièrement des cellules Leu-3,

- il se manifeste par une activité de réverse transcriptase dépendante de la présence d'ions $Mg^{2+}$, cette réverse transcriptase possédant une forte affinité pour des poly-(adénylate-oligodéoxy thymidilate) $/poly(A)$-oligo$(dT)_{12-18}/$

- il a un diamètre moyen de 139 nanomètres et il possède un noyau ayant un diamètre excentrique moyen de 41 nanomètres,

- il induit la production dans les cellules qu'il infecte d'une protéine virale p25, ayant un poids moléculaire de l'ordre de 25 000, cette protéine p25 n'étant pas reconnue par des anticorps préalablement formés contre la protéine p24, isolée dans des conditions analogues à partir de cellules infectées par un virus du type HTLV /human T cell Leukemia virus décrit dans "Journal of National Cancer Institute", 1982, vol. 69 N° 6 par Gallo et Reitz et dans "Cold Spring Harbour Laboratories",1984, Human T cell Leukemia Lymphoma virus, par Gallo, Essex et Gross/ plus particulièrement HTLV1 et HTLV2 .

- il présente une faible homologie antigénique avec le virus de l'anémie infectieuse du cheval (VAIC), en ce que les anticorps formés contre ce dernier virus immuno-précipitent la susdite protéine p25 du virus LAV,

- il présente une densité de l'ordre de 1, 16 dans un gradient de sucrose.

Il a également été montré dans l'article mentionné plus haut que le virus pouvait être propagé dans des cultures de lymphocytes T provenant de préférence d'un donneur adulte sain ou d'un cordon ombilical. Les conditions dans lesquelles les lymphocytes peuvent être infectés ont été décrites dans l'article sus-mentionné. Avantageusement cette infection se fait en présence d'un sérum anti-$\alpha$-interféron humain. La production de virus, appréciée grâce à son activité réverse-transcriptase, débute en général entre le 9ème et le 15ème jour suivant la date de l'infection et se poursuit pendant une durée de 10 à 15 jours.

Des échantillons représentatifs des deux rétrovirus LAV ont été déposés à la CNCM le 15 septembre 1983 sous les Nos 1-240 et 1-241 respectivement.

Ceci étant, une des difficultés qui n'a pu être surmontée jusqu'à ce jour réside dans la difficulté de répliquer le rétrovirus LAV en quantités importantes dans des lymphocytes T. En effet aucune lignée permanente de cellules productrices en continu du virus LAV n'a été isolée. La production de virus LAV n'est que transitoire et ceux-ci finissent par tuer l'hôte cellulaire, de sorte que la production de nouvelles doses de virus est chaque fois tributaire de cultures fraîches de lymphoctyes T. Enfin la réplication efficace _in vitro_ du virus implique la présence dans le milieu de culture d'un facteur de croissance coûteux, connu sous la désignation TCGF (abréviation de l'expression anglaise "T cell growth factor") ou également appelé interleukine II.

L'invention a pour but de remédier à ces dificultés, en particulier de fournir des lignées cellulaires à la fois permanentes  directement infectables par un rétrovirus possèdant les caractéristiques immunologiques essentielles du virus LAV et possèdant en outre la capacité d'infecter directement lesdites lignées cellulaires
et,par conséquent,de permettre la production importante de
ce nouveau rétrovirus, en vue d'études scientifiques et
médicales complémentaires et, en particulier, de la mise
au point de nécessaires ou "kits" de diagnostics in
vitro dans des conditions économiques acceptables.

L'invention découle des découvertes que le rétrovirus LAV n'a pas d'effet cytopathcgène notable à l'égard
des lymphocytes B et que les lymphocytes B peuvent être
transformés en lignées cellulaires permanentes susceptibles d'être infectées par un virus ci-après dénommé B-LAV,
et de produire ce virus qui présente les caractéristiques
antigéniques essentielles du virus LAV, dans des cultures
en suspension, et ce en l'absence du facteur de croissance
TCGF.

L'invention concerne plus particulièrement des
lignées continues de cellules lymphoblastoïdes B caractérisées par leur capacité à être infectées par le virus
ou par un isolat de ce rétrovirus et à répliquer ce rétrovirus de façon continue. Plus particulièrement encore
elles sont directement infectables par un virus ou isolat
de virus ayant les caractéristiques antigéniques essentielles du rétrovirus LAV, ce rétrovirus ayant au préalable été adapté à des lymphocytes B,éventuellement en
présence de lymphocytes T. Plus particulièrement, l'invention concerne des lignées permanentes de cellules lymphoblastoïdes B,c'ont le patrimoine génétique comprend au
moins une partie de celui du virus d'Epstein-Barr (EBV).
Mais l'invention n'est pas limitée à ce type de lignée
de cellules lymphoblastoïdes B. D'autres lignées peuvent
être utilisées, par exemple des lignées de cellules dérivées du lymphome de Burkitt, lesquelles peuvent être
rendues capables de répliquer le susdit rétrovirus.

L'invention concerne également un procédé de production desdites lignées continues de cellules lymphoblastoïdes B aptes à produire en continu le susdit rétrovirus, ce procédé étant caractérisé par le fait que ces lignées continues sont infectées avec un rétrovirus LAV préalablement adapté à la fois à des lymphocytes T et à des lymphocytes B.

Des lignées continues de cellules lymphoblastoïdes B particulièrement préférées sont celles qui ont été obtenues par la culture de lymphocytes B, éventuellement en présence de lymphocytes T, en présence d'un agent de transformation blastique et d'une souche de rétrovirus LAV, infectante pour des lymphocytes T. Avantageusement l'agent de transformation blastique est constitué par le virus d'Epstein-Barr (EBV). On obtient ainsi des lignées contirues de cellules lymphoblastoïdes B, rendues capables de répliquer un rétrovirus ayant les caractéristiques antigéniques essentielles du virus LAV. De surcroît ces lignées se sont révélées non-tumorigènes, notamment à l'occasion de  leur inoculation à des souris NUDE.

Dans une forme de mise en oeuvre du procédé selon l'invention pour produire des lignées continues de cellules de lymphoblastoïdes B, on réalise une co-culture de lymphocytes B et de lymphocytes T, d'un même donneur, en présence du virus EBV dans des conditions autorisant l'infection des lymphocytes B par le virus EBV, de préférence en présence encore d'un activateur favorisant la transformation blastique des lymphocytes B, et en présence de virus LAV dans des conditions autorisant normalement l'infection des lymphocytes T.

Un activateur préféré est constitué par la protéine A de Staphylococcus Aureus. Naturellement tout autre type d'activateur conduisant aux mêmes résultats peut être utilisé. La protéine A peut être remplacée par d'autres mitogènes, par exemple des lectines, l'agent mitogène tel que celui connu sous l'expression "Pook-weed mitogen".

Bien entendu l'invention n'est pas limitée à l'utilisation de EBV pour induire la transformation des lymphocytes B en lignées lymphablastoïdes B "immortelles", en tous les cas capables de se reproduire à travers un grand nombre de générations (au moins 60 générations) dans un milieu de culture approprié. L' EBV peut être remplacé par d'autres ADNs transformants oncogènes, tels que les gènes connus sous les désignations "Myc" "Erb", etc..

De préférence l'infection des lymphocytes B par l'agent blastique approprié, en présence des lymphocytes T, pour transformer les premiers en une lignée de cellules lymphoblastoïdes, précède la réalisation d'au moins une opération d'infection du mélange avec une souche de virus LAV.

Les lymphocytes B mis en oeuvre dans le procédé selon l'invention peuvent être constitués par tout type de cellules lymphoïdes B originaires du sang ou de tissus lymphoïdes contenant des lymphocytes B, par exemple ceux obtenus à partir de moelle osseuse, de la rate, des ganclions lymphatiques, etc.

Dans ce qui précède il a également été indiqué que les lymphocytes B doivent de préférence provenir du même donneur que les lymphocytes T ayant servi à propager le virus. L'adaptation du virus LAV au lymphocyte B est réalisée par propagation en plusieurs passages, de préférence de 8 à 10 passages du virus LAV dans des cultures successives de lymphocytes sains B et T en mélange, obtenus à partir du même individu sain.

Des caractéristiques supplémentaires de l'invention apparaîtront encore au cours de la description qui suit de réalisations particulières de lignées de cellules lymphoblastoïdes B productrices d'un rétrovirus ayant les propriétés antigéniques du rétrovirus LAV, même s'il diffère de ce dernier par un certain nombre de propriétés nouvellement acquises et dont il sera question plus loin.

L'invention concerne enfin les ratrovirus adaptés B-LAV qui peuvent être obtenus par le procédé selon l'invention, ces rétrovirus possèdant, outre les propriétés antigèniques caractéristiques du rétrovirus LAV, la capacité d'infecter des lignées continues de cellules lymphoblastoïdes B résultant notamment de la transformation blastique dans les conditions telles que décrites plus haut,
de lymphocytes B préalablement adaptés à des lymphocytes T,
en présence d'un agent de transformation blastique tel que
le virus EBV ou encore des lignées continues de cellules
d'autres lymphomes B, tels que le lymphome de Burkitt. Des
souches virales répondant à ces caractéristiques ont été
déposées à la collection nationale des cultures de microorganismes (CNCM) de l'Institut Pasteur de Paris sous les
mêmes numéros et aux dates qui seront indiquées plus loin.

Certaines souches virales préférées de l'invention se
caractérisent également par une densité un peu plus élevée
que celle des souches de virus LAV décrites par F. BARRE
SINOUSSI et Coll, notamment de l'ordre de 1,18 dans un
gradient de sucrose.

L'intérêt de disposer de telles souches est manifeste.Il permet l'infection directe de lignées continues
de lymphoblastoïdes B en vue de la production de quantités
importantes de virus. Ceci constitue la matière première
pour la production d'extraits de rétrovirus reconnaissables par des sérums de patients affectés par le SIDA ou
le SLA, notamment en vue du diagnostic in vitro de ces
affections, par exemple dans les conditions qui seront
indiquées plus loin à titre d'exemple. A ce titre l'invention concerne plus particulièrement les extraits de ces
rétrovirus qui contiennent la protéine p25, directement
reconnaissable par les sérums des susdits patients. L'invention s'étend naturellement également aux nécessaires ou
"kits" pour le diagnostic in vitro des susdites affections,
comprenant à titre de constituant principal des extraits de
rétrovirus B-LAV.

Des caractéristiques supplémentaires de l'invention
apparaîtront encore au cours de la discussion qui suit des
techniques qui peuvent être mises en oeuvre pour accéder
aussi bien à des lignées de cellules lymphoblastoïdes B
qui peuvent être rendues capables de produire en continu un virus ayant les caractéristiques antigèniques du rétrovirus LAV ainsi que les souches virales en question.

Le virus isolé à partir de lymphocytes T d'un patient atteint de lymphadénopathie fut d'abord propagé dans des lymphocytes de cordon ombilical, puis dans des lymphocytes de plusieurs donneurs adultes. Il avait au préalable été vérifié que les sérums de ces donneurs ne contenaient pas d'anticorps actifs contre le virus LAV et que leurs lymphocytes n'étaient pas aptes à libérer spontanément du virus LAV après activation, dans les conditions qui ont été décrites par BARRE-SINOUSSI et Coll.

Les lymphocytes d'un donneur particulier désigné par les initiales FR sont choisis en raison de leur capacité à produire après infection des quantités élevées et régulières de virus LAV. Mais comme indiqué plus haut la production de virus LAV était dans tous les cas temporaire, puisque suivie par une réduction, puis une interruption de la production de virus et finalement de la mort des cultures cellulaires.

La production de lignées de cellules lymphoblastoïdes B susceptibles de produire le virus de façon continue dans des cultures en suspension, a été effectuée dans les conditions suivantes. Il est entendu que les conditions de culture, puis d'infection, correspondent à celles qui ont été indiquées dans l'article de S. BARRE SINOUSSI et Coll. mentionné plus haut. Les lymphocytes complets (lymphocytes B et T en mélange) ont été d'abord stimulés pendant 24 heures avec de la protéine A, puis infectés avec une souche de virus EBV, dérivée d'un carcinome nasopharyngé (souche M 81 décrit dans B.J.A.B B 95-8 Int J. Cancer 1976 vol.17 p.161). Cinq jours plus tard, la moitié de la culture a été infectée avec le virus LAV dans les conditions décrites par BARRE SINOUSSI et Coll. puis divisée en deux sous-cultures $C_1$ et $C_2$.

La culture des cellules provenant de la sous-culture $C_1$ fut introduite dans un milieu dépourvu d'un facteur de croissance spécifique des lymphocytes T, en par-

0165120

ticulier du facteur TCGF. Au contraire les cellules de la
sous-culture $C_2$ furent cultivées dans un milieu contenant
le facteur TCGF.

Les cultures furent maintenues par addition de
milieu tous les trois jours et changement complet de milieu
chaque semaine. Comme prévu, les cultures stimulées par le
facteur de croissance TCGF produisirent du virus LAV détecté
grâce à l'apparition d'une activité de transcriptase réverse
dans le milieu entre le 12ème jour (c'est à dire le 6ème
jour après le début de l'infection par le virus LAV) et le
21ème jour. Au contraire aucune activité de transcriptase
réverse ne fut observée dans les cultures des cellules provenant de la sous-culture $C_1$ en l'absence du facteur TCGF.
Il était ainsi clairement établi qu'à ce stade le virus
LAV n'avait été produit en quantité détectable que par les
lymphocytes T activés et en présence du facteur de croissance TCGF, et ce en l'absence de production détectable
de virus par les lymphocytes B.

Le 19ème jour, au moment de l'apparition d'un déclin
de la production du virus LAV, une partie $C'_2$ de la culture
provenant de la sous-culture $C_2$, fut mise en contact avec
des lymphocytes T d'une culture fraîche de lymphocytes T
qui avait été obtenue à partir du même donneur et qui avait
été activée au préalable pendant trois jours avec de la phytohémaglutinine,et ce dans le but de fournir des cibles nouvelles pour le virus et relancer l'infection virale. Huit
jours plus tard (25ème jour) on observe effectivement l'apparition d'un nouveau type de transcriptase réverse. Cependant contrairement aux observations faites au cours de la
première infection, la production ne s'arrêta plus. Après
certaines variations quantitatives de la production au
cours du temps on observa un accroissement et une stabilisation de l'activité transcriptase réverse (supérieure à
100 000 coups par minute/ml surnageant). Le virus produit
fut ensuite extrait à partir des cultures cellulaires dans
les conditions décrites dans l'article de BARRE SINOUSSI et
Coll. mentionné plus haut.

On notera qu'au moment de la relance de l'infection par le virus LAV ,l'apparition de cellules transformées par EBV et de plus grandes dimensions avait été observée tant dans les cultures issues des sous-cultures $C_1$ que dans celles issues des sous-cultures $C_2$. Cette observation témoignait de ce que "l'immortalisation" des cellules B sous l'action de EBV s'était déjà produite. Il restait à déterminer si la production continue de rétrovirus LAV (ou d'un rétrovirus en possédant les caractéristiques antigéniques essentielles) pouvait être attribuée à une survie prolongée des cellules infectées T en présence de la lignée de cellules lymphoblastoïdes obtenues par transformation des lymphocytes B par EBV, ou au contraire à ces dernières.

C'est pour faire la discrimination nécessaire entre ces deux possibilités que la culture témoin des cellules de la sous-culture $C_1$ fut à son tour infectée par le virus obtenu à partir de la culture $C'_2$ (en fait à partir du surnageant congelé au jour 25 de la culture $C'_2$) et maintenue en l'absence de TCGF. Dans un tel milieu les population de cellules T éventuellement présentes ne pouvaient que décliner rapidement, ce qui fut d'ailleurs vérifié par l'absence de réaction avec des anticorps monoclonaux commercialisés sous la marque OKT4 par la société ORTHO, cette absence de réaction témoignant donc de l'absence d'un phénotype caractérisque des lymphocytes T. En conséquence la sous-culture productrice du virus était essentiellement constituée de cellules lymphoblastoïdes B.

Il peut raisonnablement être inféré des observations précédentes que la nouvelle propriété acquise par les virus produits par la sous-culture $C'_2$ a résulté de l'adaptation du virus aux lymphocytes B contenus dans le mélange de lymphocytes qui avait été obtenu à partir du donneur FR, avant la réalisation des essais de co-infection .En effet il a été observé que des souches de virus LAV obtenues après les tout premiers passages dans les lymphocytes de FR n'étaient pas aptes à infecter la lignée de lymphoblastoïde B issue de la sous-culture C. Au contraire la culture virale LAV obtenue après 10 passages sur le mélange de lymphocytes de FR est apte à réaliser la susdite infection. D'autres lignées cellulaires de cellules lymphoblastoïdes B ont pu être infectées dans des conditions semblables . Il est vrai que l'infection n'a pu être réalisée dans toutes les lignées de cellules lymphoblastoïdes B testées. Le tableau I qui suit témoigne cependant de ce que sur 14 lignées testées, 5 se sont révélées productrices. Il en découle pour l'homme du métier la conclusion, naturelle dans ce domaine, que l'obtention d'une production continue de virus à partir d'au moins une lignée de cellules lymphoblastoïdesB, soit naturelle, soit induite à partir de lymphocytes B dans les conditions qui ont été indiquées plus haut, n'impliquera des essais d'infection que sur un nombre limité de lignées de cellules lymphoblastoïdes B, soit induites à partir de lymphocytes B, soit "naturelles". La nécessité de répéter les essais d'infection sur des lignées différentes en nombre limité relève de la routine pour le spécialiste du domaine technique concerné.

C'est ce dont témoignent les résultats apparaissant dans le tableau et obtenus par des essais d'infection in vitro avec LAV B, de différentes lignées cellulaires de lymphoblastoïdes B produites à partir de lymphocytes obtenus à partir d'adultes ou de cordons ombilicaux préalablement infectés avec des souches de EBV NPC (M 81) ou (B 95/8). Les résultats témoignent de ce que des productions de virus LAV ont été obtenues à partir de cellules lymphoblastoïdes B obtenues à partir d'une culture de lymphocytes de cordon ombilical (LCo), à partir de lymphocytes d'un autre donneur BAR (c'est à dire à partir de cultures de lymphocytes issus de deux donneurs sur quatre). Il est à remarquer que le virus B-LAV dérivé de la lignée cellulaire lymphoblastoïde B du donneur FR est apparu plus rapidement et sous un titre plus élevé, ce résultat suggérant que la souche virale B-LAV avait bien subi une adaptation secondaire aux cellules B, après son passage dans le mélange initial de lymphocytes de FR.

D'autres lignées cellulaires obtenues à partir

de cellules lymphoblastoïdes B (naturelles), notamment de lignées cancéreuses présentant certains des phénotypes des lymphocytes B, ont également été testées pour leur susceptibilité à l'infection par les virus LAV et B-LAV. Seule la souche BJAB (non infectée au préalable par EBV) a été infectée par B-LAV après une période prolongée de 30 jours. Au contraire la même souche n'a pas pu être infectée dans les mêmes conditions par la souche LAV initiale. Cependant,la transformation in vitro préalable de la même lignée cellulaire avec la souche B 95/8 de EBV a permis une potentialisation de la production ultérieure par la même lignée de virus LAV-B. En effet on a constaté un rendement de production de virus B-LAV de 2 à 3 fois plus élevée que dans le cas de la lignée BJAB décrite dans Biomedicine 1975, vol. 2 22, p. 276, non préalablement transformée par EBV.

Certes le tableau fait apparaître que d'autres lignées cellulaires "naturelles" n'ont pu être affectées. Mais il est tout à fait remarquable que celles des lignées cellulaires qui se sont révélées produire le virus B-LAV le faisaient en l'absence de tout facteur de croissance, qu'il soit spécifique des lymphocytes T ou des lymphocytes B.

Les résultats sont résumés dans le tableau qui suit :

13

TABLEAU

| Lignées de cellules | Origine | Présence du génome EBV | Retro-virus LAV | Croissance de B-LAV | CNCM |
|---|---|---|---|---|---|
| FR 8 | lymphocytes B d'un donneur adulte | oui | ++ | ++ | I-303 |
| BAR | " " | oui | + | ++ | |
| BRA | " " | oui | − | − | |
| DIER | " " | oui | − | − | |
| LCo | lymphocyte B de cordon ombilical | oui | + | ++ | I-302 |
| LC1 | " " | oui | − | − | |
| Daudi | lymphome de Burkitt | oui | − | − | |
| Namalwa | " " | oui | − | − | |
| Raji | " " | oui | − | − | |
| Chev | " " | oui | − | − | |
| BJAB | " " | non | − | + | I-300 |
| BJAB/B95/8 | " " | oui | − | ++ | I-301 |
| MOLT/4 | T-lymphome | non | − | − | |
| HL60 | Leucémie myéloïde | non | − | − | |

Les lignées cellulaires suivantes ont fait l'objet de dépôts à la CNCM, le 9 mai 1984 sous les Nos sus-indiqués. Le virus B-LAV lui-même a été déposé à la CNCM à la même date sous le N° I-299.

Les souches de virus B-LAV peuvent être isolées à partir des surnageants des cultures cellulaires qui l'ont reproduit et peuvent ensuite être à nouveau propagées dans d'autres lignées cellulaires de cellules lymphoblastoïdes B, dès lors que leur capacité à produire le virus aura été reconnue. Bien que le B-LAV ne montre pas d'effets cytopathogènes sur les lignées du type B, il est préférable d'ajouter périodiquement environ 50% de nouvelles cellules non infectées quand la production virale commence à décliner (environ tous les deux mois).

14

L'analyse des caractéristiques morphologiques
et immunologiques des souches B-LAV a conduit à la conclusion qu'elles possèdent toutes les caractéristiques des
souches LAV initialement mises en oeuvre et qui ont été
définies dans l'article de S. BARRE SINOUSSI et Coll. Elles
présentent en outre la caractéristique de pouvoir infecter
les susdites lignees de cellules lymphoblastoïdes B. Certaines de
ces souches, en particulier celles obtenues à partir du
donneur FR 8 se sont révélées présenter une densité un peu
plus élevée que la souche initiale (1,18 au lieu de 1, 17
dans un gradient de glucose ).

L'examen plus détaillé des lignées productrices
de cellules lymphoblastoïdes B confirme l'appartenance aux
lignées B. Ces cellules présentent les marqueurs caractéristiques des lymphocytes B (immunoglobulines specifiques
présentes à la surface). Les cellules obtenues à partir de
lymphocytes B infectés avec EBV se sont toutes révélées
posséder l'antigène EBNA (abréviation de l'expression
anglaise "Epstein-Barr nuclear antigen"). Une expression de
l'antigène précoce (EA) a pu être observée dans 0,1% des
cellules de la lignée LCo et dans 3,5% des lignées FR 8.
Aucune des cellules  de la lignée NCo n'a produit de
réponse positive dans le test de détection de l'antigène de
capside virale. Cet antigène était cependant présent dans
1,5% des cellules de la lignée FR 8.

En ce qui concerne enfin les différentes préparations de virus B-LAV, il a été constaté que leurs acides
nucléiques respectifs ne conduisaient à aucune hybridation
avec des sondes d'ADN contenant le fragment G codant pour
l'antigène précoce EA (abréviation de l'expression anglaise
"early antigen") dans les essais réalisés sur filtre de cellulose. Il en résulte que les préparations obtenues de
rétrovirus ne témoignaient pas d'une contamination détectable
par le génome du virus EBV.

L'invention concerne  enfin un procédé de diagnostic in vitro  de SLA ou de SIDA, ce procédé comprenant la mise en contact d'un sérum ou autre milieu biologique provenant d'un patient présumé pouvoir être affecté et la détection de la réaction immunologique éventuellement produite.

Des modes de mise en oeuvre préférés de ce procédé mettent en jeu des essais immunoenzymatiques ou immuno-fluorescents, notamment selon la technique ELISA. En conséquence l'invention est également relative à des extraits viraux pourvus d'un marqueur, par exemple un marqueur enzymatique, fluorescent, radioactif, etc.

Les essais de diagnostic pourront comprendre les opérations suivantes :

- dépôts de quantités déterminées d'un extrait conforme à l'invention dans les godets  d'une microplaque pour titration;

- introduction dans ces godets de dilutions croissantes du sérum soumis au diagnostic;

- incubation de la microplaque;

- lavages poussés  de la microplaque;

- introduction dans les godets de la microplaque d'anticorps marqués et dirigés contre des immunoglobulines de sang, le marquage étant de préférence réalisé par une enzyme susceptible d'hydrolyser un substrat , l'hydrolyse mettant  en jeu une modification de l'absorption par ledit substrat de certaines bandes de longueur d'ondes déterminées et

- détection, de préférence par comparaison avec des témoins, de la quantité de substrat hydrolysé, la mesure obtenue conduisant à une appréciation du risque auquel est exposé le patient ou de la présence effective de la maladie.

En conséquence l'invention concerne également des nécessaires ou "kits" pour réaliser le susdit diagnostic in vitro, ces nécessaires comprenant :

- un extrait ou une fraction davantage purifiée du virus B-LAV, cet extrait et cette fraction étant marqués, par exemple par un marqueur radioactif, enzymatique, ou immunofluorescent;

- des anti-immunoglobulines humaines ou de la protéine A (avantageusement fixées sur un support insoluble dans l'eau, par exemple des billes d'agarose);

- un extrait lymphocytaire obtenu à partir d'un donneur sain;

- des tampons et si nécessaire des substrats pour permettre la visualisation du marqueur.

On décrira encore ci-après à titre d'exemple seulement, des constitutions préférées des réactifs pouvant être utilisés pour le diagnostic in vitro et les conditions de leur mise en oeuvre.

Principe de la méthode

Ce test ELISA est mis en route pour la détection et le titrage des anticorps sériques anti-rétrovirus type LAV.

Il est réalisé par un test de compétition entre un antigène viral (cultivé sur cellules lymphoblastoïdes B) et un antigène témoin constitué par un lysat des mêmes cellules non infectées.

La fixation des anticorps sur les deux antigènes est révélée par l'utilisation d'une antiglobuline humaine marquée par une enzyme, elle-même révélée par l'addition d'un substrat correspondant.

Préparation d'antigène viral

Les cultures cellulaires utilisées sont des cellules lymphoblastoïdes B d'origine humaine, infectées avec B-LAV dans les conditions sus-indiquées.

On utilise le surnageant des cellules infectées par le virus. Il est concentré par précipitation au PEG 10%, purifié successivement sur gradients de saccharose (20 à 60°) par ultracentrifugation à l'équilibre.

Les fractions virales sont récoltées et concentrées par centrifugation = 50.000 RPM pendant 60 minutes.

Le culot des virus est repris dans un volume minimum de tampon NTE pH = 7,4 (Tris 0,015 M -NaCl 0,1M - EDTA 1 milli-Molaire).

La concentration protéique est déterminée par la méthode de Lowry.

Le virus est ensuite lysé par un tampon RIPA + SDS (0,5% final) pendant 15 minutes à 37°C. Le tampon RIPA est décrit dans Sciences 1983, vol. 220, page 868 - 871. On peut utiliser pour la lyse du virus la variante suivante : solution de 1% de TRITON x 100 et de 0,1% de désoxycholate de sodium en tampon RIPA, préparée sans SDS. la méthode de Lowry.

Le virus est ensuite lysé par un tampon RIPA + SDS (0,5% final) pendant 15 minutes à 37°C.

<u>Préparation de l'antigène témoin</u>

Les cellules lymphoblastoïdes B en culture, mais non infectées, selon les conditions précédentes depuis 5 à 10 jours, sont centrifugées à faible vitesse et lysées dans le tampon RIPA en présence de 5 % du composé commercialisé sous la marque ZYMOFREN (Spécial) soit 500 U/ml. Après un séjour de 15 minutes à 4°C avec fréquentes agitations dans le dispositif d'agitation violente connu sous la désignation VORTEX, le lysat est centrifugé à 10.000 tours/minute. Le surnageant constitue l'antigène témoin. On mesure sa concentration en protéines par la méthode de Lowry.

<u>Réactifs</u>

1 - Plaques = NUNC - sp ciales ELISA contrôlées

2 - Tampon PBS : pH = 7,5

3 - Agent mouillant commercialisé sous la marque TWEEN 20

4 - Tampon carbonate : pH = 9,6 $\begin{cases} CO_3NA_2 = 0,2\ M \\ CO_3HN_a = 0,2\ M \end{cases}$

5 - Sérum de veau non foetal : conservé congelé (Biopro)

6 - Sérum d'albumine bovine (BSA) SIGMA (fraction V)

7 - Anti IgG humaines (H + L) marquées à la peroxydase de l'Institut Pasteur = flacons de 1 ml - conservation à +4°C

8 - Tampon de lavage = tampon PBS pH 7,5 + 0,05% TWEEN 20

Dilution du conjugué = à la dilution indiquée en tampon
PBS + TWEEN 20 (0,05%) + Albumine bovine 0,5 g pour 100 ml.
9 - Tampon dilution des sérums = tampon PBS + 0,05% TWEEN 20
10- Substrat = EPO

* tampon citrate pH = 5,6

$\begin{bmatrix} \text{citrate trisodique } (C_6H_5Na_4O_3, 2H_2O) \text{ (Merk)} \\ \qquad\qquad\qquad\qquad\qquad\qquad 0,05 \text{ M} \\ \text{acide citrique } (C_6H_8O_7, 1H_2O) \text{ (Merk) } 0,05 \text{ M} \end{bmatrix}$

*, eau oxygénée = à 30% (110 volumes) - utilisée à 0,03% au moment
de l'emploi en tampon citrate.

* ortho-phénylène-diamine : SIGMA.
75 mg pour 25 ml tampon - A diluer en tampon citrate extemporanément.

Préparation des plaques

On utilise des plaques 96 godets à fond U (NUNC =
ELISA). Elles comprennent 12 colonnes, respectivement de 8
godets, numérotées de 1 à 12.

La répartition des antigènes est faite dans les
conditions suivantes :
- 100 μl de l'antigène viral dilué en tampon carbonate pH = 9,6
  sont déposés dans chaque godet des colonnes :
       1 - 2 - 5 - 6 - 9 - 10
  Les godets de ces colonnes sont ci-après désignés sous
sous l'expression "godets +".
- De même 100 μl de l'antigène témoin dilué en tampon carbonate pH = 9, 6 sont déposés dans les godets des colonnes :
       3 - 4 - 7 - 8 - 11 - 12
  Les godets de ces dernières colonnes sont ci-après
  désignés par l'expression "godets -".

La dilution de l'antigène viral est titrée à chaque
production virale. Plusieurs dilutions d'antigène viral sont
testées en fonction de témoins positifs et négatifs connus
(à plusieurs dilutions) et en fonction de l'anti-IgG humaines, marquées à la peroxydase, testées aussi à plusieurs
dilutions.

En règle générale , la concentration protéique de
la préparation est de 5 à 2,5 μg/ml.

La même concentration protéique est utilisée pour l'antigène témoin.

Les plaques sont fermées à l'aide d'un couvercle de plastique et sont incubées une nuit à +4°C.

Elles sont ensuite mises une fois en eau distillée puis bien essorées. Les godets sont alors remplis de 300 µl de sérum de veau foetal à 20% en tampon PBS.

L'incubation dure 2 heures à 37°C (plaques couvertes).

Les plaques sont lavées 3 fois en tampon PBS + TWEEN 20, 0,05% = PBS-tw.

. 1er lavage 300 µl

. 2ème et 3ème lavages 200 µl/godet.

Les plaques essorées, puis scellées avec film de plastique adhésif peuvent être conservées à -80°C.

Réaction ELISA = Recherche en titrage d'anticorps :

Après décongélation, les plaques sont lavées 3 fois en PBS- tw . Elles sont essorées et séchées.

Les sérums témoins positifs, négatifs, ainsi que les sérums testés sont dilués en tubes au préalable, en PBS-tw 0,05% Albumine bovine à 0,5%.

La dilution choisie est le 1/40.

- 100 µl de chaque sérum sont déposés en double sur l'antigène viral et en double sur l'antigène témoin.

- de même pour les sérums positifs et négatifs dilués.

- 100 µl de PBS + TWEEN + albumine bovine sont déposés de même dans 2 "godets + " et 2"godets - " pour constituer le témoin conjugué.

Les plaques munies de leur couvercle sont incubées 1h30 à 37°C. Elles sont lavées 4 fois en PBS = TWEEN 0,05% - 100 µl d'anti-IgG humaines (marquées à la péroxydase) à la dilution choisie, sont déposées dans chaque godet et incubés à 37°C.

Les plaques sont à nouveau lavées 5 fois au PBS + TWEEN. Elles sont essorées et séchées.

La révélation de la réaction enzymatique sera effectuée à l'aide d'un substrat ortho-phénylène-diamine (0,05% en tampon citrate pH 5,6 contenant 0,03% $H_2O_2$).

100 ul de substrat sont répartis dans chaque godet.

Les plaques sont mises au noir 20 minutes à la température du laboratoire.

Lecture au spectrophotomètre (pour microplaques à 492 nm.

Sont considérés comme des sérums possèdant des anticorps contre ce virus ceux auxquels correspondent des différences de densité optique (entre la densité optique de l'antigène viral et la densité optique de l'antigène témoin) supérieures ou égales à 0,30%.

Cette technique permet un dosage qualitatif, mais aussi quantitatif. Pour cela, on peut, soit effectuer des dilutions du sérum à tester, soit comparer une dilution du sérum à une gamme de témoins testés, dans les mêmes conditions.

0165120

REVENDICATIONS

1   Lignées continues de cellules lymphoblastoïdes B
caractérisées par leur capacité à produire en continu un
rétrovirus ayant les caractéristiques antigéniques essentielles du virus LAV, lorsqu'elles ont au préalable été infectées
avec le virus ou avec un isolat de ce rétrovirus.

2 - Lignées de cellules lymphoblastoïdes B continues,
caractérisées en ce qu'elles sont directement infectables
par un virus ou isolat de virus ayant les caractéristiques
antigéniques essentielles du rétrovirus LAV, ce rétrovirus
ayant au préalable été adapté à des lymphocytes B en présence de lymphocytes T.

3 - Lignées selon la revendication 1 ou la revendication 2, caractérisées par leur absence de tumorigénicité
dans des souris NUDE.

4 - Lignées selon l'une quelconque des revendications 1 à 3, caractérisées par le fait qu'elles comportent au
moins une partie du patrimoine génétique du virus d'Epstein
Barr.

5 - Lignées selon la revendication 1 ou la revendication 2, caractérisées par le fait qu'elles sont dérivées
d'un lymphome de Burkitt.

6 - Procédé de production de lignées continues de
cellules lymphoblastoïdes B, aptes à produire en continu
un rétrovirus ayant les caractéristiques antigéniques essentielles du rétrovirus LAV, caractérisé par le fait que
ces lignées continues sont infectées avec un rétrovirus
B-LAV préalablement adapté à la fois à des lymphocytes T
et à des lymphocytes B.

7 - Procédé selon la revendication 8 caractérisé par
le fait que les lignées continues de cellules lymphoblastoïdes B mises en oeuvre ont été obtenues par la culture de
lymphocytes B préalablement adaptés à des lymphocytes T,
en présence d'un agent de transformation blastique des
lymphoctyes B et d'une souche de rétrovirus LAV infectante
pour les lymphocytes T.

8 - Procédé selon la revendication 6 ou selon la revendication 7 caractérisé en ce que l'agent de transformation blastique est constitué par le virus d'Epstein-Barr.

9 - Procédé selon la revendication 7 ou la revendication 8 caractérisé en ce que l'infection des lymphocytes B par l'agent blastique a précédé l'infection des lymphocytes T par le virus LAV.

10 - Procédé selon la revendication 6 caractérisé en ce que la lignée continue de cellules lymphoblastoïdes B est constituée par une lignée de cellules d'un lymphome de Burkitt, le cas échéant lui-même transformé par le génome du virus de Epstein-Barr.

11 - Procédé selon l'une quelconque des revendications 6 à 10, caractérisé en ce que les lignées continues de cellules de lymphoblastoïdes B ont été obtenues par co-culture de lymphocytes B adaptés aux lymphocytes T, d'une part, et de lymphocytes T, d'autre part, et en présence du virus de Epstein-Barr dans des conditions autorisant l'infection des lymphocytes B et en présence encore d'un activateur favorisant la transformation blastique des lymphocytes B.

12 - Procédé selon la revendication 11 caractérisé en ce que la susdite co-culture est réalisée en présence d'un activateur, tel qu'une protéine A de Staphylococcus aureus .

13 - Rétrovirus ou isolat de ce rétrovirus, qui ont les propriétés antigéniques essentielles d'un rétrovirus T-lymphotropique, isolable à partir de sérums de patients affectés par le syndrome d'immunodéficience acquise (SIDA) ou par un syndrome mettant en jeu des lymphadénopathies (SLA), ce rétrovirus possédant l'ensemble des propriétés biologiques suivantes :

- les cibles préférées de ce rétrovirus sont constituées par des lymphocytes T, notamment des cellules Leu-3,
- il se manifeste par une activité de réverse transcriptase dépendante de la présence d'ions $Mg^{2+}$, cette réserve transcriptase possédant une forte affinité pour des poly-(adénylate-oligodéoxy-thymidylate) $/poly(A)-oligo(dT)_{12-18}/$

- il a un diamètre excentrique de 139 nanomètres et il possède un noyau ayant un diamètre moyen de 41 nanomètres,

- il induit la production dans les cellules qu'il infecte d'une protéine virale p25, ayant un poids moléculaire de l'ordre de 25 000, cette protéine p25 n'étant pas reconnue par des anticorps préalablement formés contre la protéine p24, isolée dans des conditions analogues à partir de cellules infectées par un virus du type HTLV,

- il présente une faible homologie antigénique avec le virus de l'anémie infectieuse du cheval (VAIC), en ce que les anticorps formés contre ce dernier virus immuno-précipite la susdite protéine p25 du virus LAV,

ce rétrovirus étant plus particulièrement caractérisé par son adpatation aux lymphocytes B et par sa capacité à infecter des lignées continues de cellules lymphoblastoïdes B, telles que celles résultant de la transformation blastique des lymphocytes B, préalablement adaptés à des lymphocytes T, en présence d'un agent de transformation blastique tel que le virus d'Epstein-Barr, ou des lignées continues de cellules d'un lymphome de Burkitt.

14 - Rétrovirus selon la revendication 13, caractérisé par sa capacité à infecter des lignées continues de lymphoblastoïdes B, du type de celles qui ont été déposées à la Collection Nationale des Cultures de Microorganismes (CNCM) de l'Institut Pasteur de Paris, sous les Nos I-300, I-301; I-302 et I-303.

15 - Rétrovirus selon la revendication 13 ou la revendication 14 caractérisé en ce qu'il présente une densité de l'ordre de 1,18 dans un gradient de saccharose

16 - Application du rétrovirus obtenue par le procédé selon l'une quelconque des revendications 1 à 12, ou du rétrovirus selon l'une quelconque des revendications 13 à 15, à la production d'extraits de rétrovirus reconnaissables par des sérums de patients affectés par le SIDA ou les SLA., notamment en vue du diagnostic in-vitro de ces affections.

17 - Extraits du rétrovirus obtenu par le procédé selon l'une quelconque des revendications 13 à 15, cet

0165120

24

extrait contenant la susdite protéine p25.

18 - Nécessaire ou "kit" pour le diagnostic <u>in</u> <u>vitro</u> du SIDA ou de LAS comprenant à titre de constituant principal un extrait conforme à la revendication 17.

Office européen
des brevets

**RAPPORT DE RECHERCHE EUROPEENNE**

**0165120**

Numero de la demande

EP  85 40 0906

## DOCUMENTS CONSIDERES COMME PERTINENTS

| Categorie | Citation du document avec indication en cas de besoin des parties pertinentes | Revendication concernee | CLASSEMENT DE LA DEMANDE (Int Cl 4) |
|---|---|---|---|
| A | PROC. NATL. ACAD. SCI., vol. 81, janvier 1984, pages 33-37, Biochemistry, US; R.D. LASKY et al.: "Possible DNA-RNA tumor virus interaction in human lymphomas: expression of retroviral proteins in Ramos lymphoma lines is enhanced after conversion with Epstein-Barr virus"<br>* Page 33, colonne de droite, lignes 19-37 *<br><br>--- | 1-11 | C 12 N 5/00<br>C 12 N 7/00<br>G 01 N 33/569<br>G 01 N 33/531 |
| A | NATURE, vol. 309, 3 mai 1984, pages 12,13; Chesham, Bucks, GB; R. WEISS: "Acquired immune deficiency syndrome: Retroviruses linked with AIDS"<br>* Page 12, colonne du milieu, ligne 15 - colonne de droite, ligne 12 *<br><br>---              -/- | 13-18 | |

DOMAINES TECHNIQUES
RECHERCHES (Int Cl 4)

C 12 N
A 61 K
G 01 N

Le présent rapport de recherche a été établi pour toutes les revendications

| Lieu de la recherche | Date d'achèvement de la recherche | Examinateur |
|---|---|---|
| LA HAYE | 16-08-1985 | RYCKEBOSCH A.O.A. |

## 0165120
Numero de la demande

**RAPPORT DE RECHERCHE EUROPEENNE**

Office européen
des brevets

EP   85 40 0906

| | DOCUMENTS CONSIDERES COMME PERTINENTS | | Page   2 |
|---|---|---|---|

| Catégorie | Citation du document avec indication en cas de besoin des parties pertinentes | Revendication concernee | CLASSEMENT DE LA DEMANDE (Int Cl 4) |
|---|---|---|---|
| A | SCIENCE, vol. 224, no. 4648, 4 mai 1984, pages 497-500; M. POPOVIC et al.: "Detection, isolation, and continuous production of cytopathic retroviruses (HTLV-III) from patients with AIDS and pre-AIDS" * Page 497, colonne de droite, ligne 60 - page 498, colonne de gauche, ligne 5; page 498, colonne du milieu, ligne 5 - colonne de droite, ligne 25; page 499, colonne de gauche, lignes 45-47 * | 1-18 | |
| | --- | | |
| D,A | SCIENCE, vol. 220, no. 4599, 20 mai 1983, pages 868-871; F. BARRE-SINOUSSI et al.: "Isolation of a T-lymphtropic retrovirus from a patient at risk for acquired immune deficiency syndrome (AIDS)" * En entier * | 1-18 | |
| | --- | | DOMAINES TECHNIQUES RECHERCHES (Int Cl 4) |
| P,X | SCIENCE, vol. 225, no. 4657, 6 juillet 1984, pages 63-66; L. MONTAGNIER et al.: "Adaption of Lymphadenopathy Associated Virus (LAV) to replication in EBV-transformed B Lymphoblastoid cell lines" * En entier * | 1-18 | |
| | ----- | | |

Le present rapport de recherche a ete etabli pour toutes les revendications

| Lieu de la recherche LA HAYE | Date d'achèvement de la recherche 16-08-1985 | Examinateur RYCKEBOSCH A.O.A. |
|---|---|---|

CATEGORIE DES DOCUMENTS CITES

X   particulièrement pertinent à lui seul
Y   particulièrement pertinent en combinaison avec un
    autre document de la même catégorie
A . arrière-plan technologique
O   divulgation non-écrite
P   document intercalaire

T :  théorie ou principe à la base de l'invention
E :  document de brevet antérieur, mais publie à la
     date de dépôt ou après cette date
D :  cité dans la demande
L :  cité pour d autres raisons

& :  membre de la même famille. document correspondant

OEB Form 1503 03 82